(19) Europäisches Patentamt
European Patent Office
Office européen des brevets

(11) **EP 3 431 118 B1**

(12) **EUROPÄISCHE PATENTSCHRIFT**

(45) Veröffentlichungstag und Bekanntmachung des Hinweises auf die Patenterteilung:
**10.04.2024   Patentblatt 2024/15**

(21) Anmeldenummer: **18183941.6**

(22) Anmeldetag: **17.07.2018**

(51) Internationale Patentklassifikation (IPC):
**A61M 1/16** (2006.01)   **A61M 1/36** (2006.01)

(52) Gemeinsame Patentklassifikation (CPC):
**A61M 1/1605; A61M 1/165; A61M 1/1656; A61M 1/3609**

(54) **VORRICHTUNG ZUR DURCHFÜHRUNG EINER ISONATRÄMISCHEN DIALYSE**

DEVICE FOR PROVIDING ISONATRAEMIC DIALYSIS

DISPOSITIF POUR LA MISE EN OEUVRE D'UNE DIALYSE ISONATRIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2017   DE 102017116097**

(43) Veröffentlichungstag der Anmeldung:
**23.01.2019   Patentblatt 2019/04**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
EP-A1- 0 407 737     WO-A1-2017/080970
DE-C1- 19 734 992

## Beschreibung

Die vorliegende Erfindung betrifft eine Vorrichtung zur extrakorporalen Blutbehandlung (z.B. Hämodialyse), wobei das Blut eines Patienten von einer sogenannten Dialysierflüssigkeit in einem Dialysator umspült wird.

## Hintergrund der Erfindung

Ziel einer Dialysetherapie ist es, neben der Entgiftung des Blutes, überschüssiges Wasser, welches sich aufgrund eines der Dialyse zugrundeliegenden Nierenversagens im Körper ansammelt, aus diesem zu entfernen. Dies geschieht durch eine sogenannte Ultrafiltration, bei der Flüssigkeit über einen Dialysator aus dem Blut entfernt wird.

## Stand der Technik

Herkömmliche Dialysatoren weisen üblicherweise ein röhrenförmiges Dialysatorgehäuse mit einer Längsausdehnung auf, wobei der Innenraum des Dialysators einen Querschnitt aufweist, der sich typischer Weise über die gesamte Längsausdehnung nicht oder nur unwesentlich verändert. Im Innenraum sind parallel zueinander angeordnet Kapillarmembranen vorgesehen. Die Kapillarmembranen bilden zusammen einen Abschnitt eines extrakorporalen Blutkreislaufes aus, während der Außenraum der Kapillaren und der Innenraum des Dialysatorgehäuses einen Abschnitt eines Kreislaufs der Dialysierflüssigkeit ausbilden. Die beiden Kreisläufe sind gegenläufig und über die semipermeablen Membranen der Kapillaren voneinander getrennt. Durch diese semipermeablen Membranen finden sowohl ein Wasser- als auch ein Stoffaustausch statt. Insbesondere werden dem Blut des Patienten Wasser und Schadstoffe entzogen. Im Durchmesser oder im Molekulargewicht größer werdende Retentionsprodukte werden bei Dialysatoren durch diffusive Prozesse über die Membranen in schlechterer Weise entzogen als kleinere Schadstoffe.

Als verschiedene Dialysetechniken werden u.a. Hämodialyse, Hämodiafiltration und High-Flux-Dialyse eingesetzt. Bei der Hämodialyse wird nach dem Prinzip des Konzentrationsausgleichs kleinmolekularer Substanzen zweier Flüssigkeiten verfahren, die durch eine semipermeable Membran getrennt sind (Diffusion). Von der Filtermembran getrennt befindet sich auf der einen Seite das Blut mit Elektrolyten wie Kalium und Phosphat sowie harnpflichtigen Substanzen (z.B. Harnstoff, Harnsäure). Auf der anderen Seite der Membran befindet sich die Dialysierflüssigkeit, welche einen an den jeweiligen Bedürfnissen des Patienten orientierten Anteil an Elektrolyten aufweist. Im Einzelnen besteht die Dialysierflüssigkeit aus hochreinem Wasser, einer ersten basischen Komponente (z.B. Natriumhydrogencarbonat (NaHCO$_3$)) sowie einer zweiten sauren Komponente. Letztere setzt sich bspw. aus Natriumchlorid (NaCl), Kaliumchlorid (KCl), Magnesiumchlorid (MgCl$_2$), Calciumchlorid (CaCl$_2$), Essigsäure (CH$_3$COOH) und Glucose zusammen. Die semipermeable Filtermembran (Dialysemembran) zwischen Blut und Dialysierflüssigkeit besitzt Poren, die kleine Moleküle wie Wasser, Elektrolyte und harnpflichtige Substanzen durchlassen, aber große Moleküle wie Eiweiße und Blutzellen zurückhalten.

In der Regel werden Dosierpumpen und Leitfähigkeitssonden zur Herstellung bzw. Proportionierung der Dialysierflüssigkeit eingesetzt. Eine Sonde misst dabei die Leitfähigkeit nach Zugabe des Natriumhydrogencarbonats mittels einer ersten Dosierpumpe. Eine weitere Sonde erfasst die Leitfähigkeit der gesamten Dialysierflüssigkeit, nachdem auch die saure Komponente mittels einer weiteren Dosierpumpe hinzugegeben worden ist. Anhand der gemessenen Leitfähigkeiten werden dann die Zugabemengen geregelt. Dieses Verfahren ist als leitfähigkeitsgesteuerte Proportionierung bekannt.

Bei der sogenannten volumetrischen Proportionierung dienen die Leitfähigkeitssonden lediglich zur Kontrolle. Die Proportionierung erfolgt hier direkt über die Dosierpumpenförderraten, was Kenntnisse über die Zusammensetzung der eingesetzten Komponenten erfordert.

Die richtige Zusammensetzung der Dialysierflüssigkeit spielt eine wichtige Rolle bei der Dialyse. Insbesondere Natrium spielt eine große Rolle, da es sowohl in der Dialysierflüssigkeit als auch im Blutplasma das am häufigsten vorkommende Kation ist. Natrium wird insbesondere in Form von Kochsalz (NaCl) aufgenommen. Eine erhöhte Natriumaufnahme kann dadurch ausgeglichen werden, dass eine entsprechende Menge Flüssigkeit zugeführt wird. Eine erhöhte Flüssigkeitsaufnahme führt allerdings dazu, dass während des nächsten Dialysevorgangs mehr überschüssige Flüssigkeit durch Ultrafiltration entzogen werden muss. Hohe Ultrafiltrationsraten machen aber wiederum eine Zugabe von Natrium erforderlich, falls es aufgrund des Flüssigkeitsentzuges zu Blutdruckabfällen kommt. Dies führt nun wieder zu einer erhöhten Wasseraufnahme. Ein Teufelskreis beginnt also. Ein zu geringer Natriumwert in der Dialysierflüssigkeit ist allerdings auch nicht vorteilhaft. Der intrazelluläre und der extrazelluläre Raum stehen in einem osmotischen Gleichgewicht zueinander. Eine geringe Natrium-Konzentration in der Dialysierflüssigkeit würde aufgrund von Diffusionsprozessen innerhalb des Dialysators zu einem Absinken der extrazellulären Osmolarität führen. Da aber beide Räume im Gleichgewicht stehen, würde Wasser in unerwünschter Weise aus dem intrazellulären Raum in den extrazellulären Raum strömen.

Aus oben genannten Gründen ist es deshalb wünschenswert, die Natrium-Konzentration in der Dialysierflüssigkeit individuell so anzupassen, dass die Plasma-Natrium-Konzentration während der Dialyse möglichst wenig und bestenfalls gar nicht geändert wird. Eine solche Dialyse wird als isonaträmisch bezeichnet (vgl. bspw. de Paula, F. M.; Peixoto, A. J.; Pinto, I. V.; Dorigo, D.; Patricio, P. J. M. and Santos, S. F. F.: "Clinical consequences of an Individualized dialysate sodium Prescription in hemodialysis patients", Kidney Internationol, 2004, 66, 1232-1238 und Basile, C. and Lomonte. C.: "It is Time to Individualize the Dialysate Sodium Prescription", Seminars in Dialysis, 2016, 29, 24-27).

Am einfachsten wäre es, vor jeder Dialyse eine Blutprobe zu entnehmen und zu analysieren, um die Natrium-Konzentration in der Dialysierflüssigkeit auf dieser Basis einzustellen. Allerdings ist diese Vorgehensweise sehr zeitintensiv und mit einem hohen apparativen Aufwand verbunden. Aus diesem Grund wird in vielen Dialysezentren mit einer standardisierten Zusammensetzung der Dialysierflüssigkeit dialysiert, obwohl die Plasma-Natrium-Konzentration von Patient zu Patient unterschiedlich ausfallen kann (vgl. Mendoza, J. M.; Sun, S.; Chertow, G. M.; Moran, J.; Doss, S. and Schiller, B.: "Dialysate Sodium and Sodium gradient in maintenance hemodialysis: a neglected sodium restriction approach?", Nephrol Dial Transplant. 2011, 26, 1281-1287).

Die vorgenannten bekannten Lösungsansätze sind dahingehend nachteilig, dass die Ermittlung der benötigten Parameter zeitaufwändig ist, dass häufig zwei Sensoren vor und hinter dem Dialysator mit entsprechenden Kalibrierungsaufwand erforderlich sind, bzw. dass Leitfähigkeitsmessungen am Ausgang der verbrauchten Dialysierflüssigkeit durch aus dem Blut in die verbrauchte Dialysierflüssigkeit gelangende Substanzen beeinflusst werden.

Weiterer Stand der Technik ist bekannt aus WO 2017/080970 A1, aus welcher eine Vorrichtung zur extrakorporalen Blutbehandlung bekannt ist, mit einer Filtereinheit, die an einen Blutkreislauf und an einen Dialysierflüssigkeitskreislauf angeschlossen ist, einer Aufbereitungseinrichtung zur Aufbereitung und Regulierung der Zusammensetzung der Dialysierflüssigkeit; und eine Steuereinheit, die dazu eingerichtet ist, einen Vorschlagswert einer Natriumkonzentration für die Dialysierflüssigkeit in der Dialysezuleitung zu ermitteln oder zu empfangen und einen Einstellwert für die Natriumkonzentration in der Dialysierflüssigkeit als Funktion des vorgeschlagenen Wertes zu bestimmen. Für mindestens ein Intervall von Vorschlagswerten für die Natriumkonzentration ist die Steuereinheit dazu konfiguriert, den Einstellwert so zu bestimmen, dass sich der Einstellwert vom Vorschlagswert unterscheidet und dass aus unterschiedlichen Vorschlagswerten unterschiedliche Sollwerte ermittelt werden. Der eingestellte Wert ist auf einen vorgegebenen Pivot-Wert ausgerichtet, der z. B. die durchschnittliche Natriumkonzentration im Blut einer Bevölkerung darstellt und nicht außerhalb eines Referenzbereichs liegen darf.

Ferner ist aus DE 197 34 992 C1 bekannt, dass eine in-vivo-Bestimmung von Parametern der Hämodialyse notwendig ist, um ein Behandlungsverfahren nach der Hämodialyse optimieren zu können. Ein solcher Wert ist insbesondere der Wert für die Austauschleistung des Dialysators, dargestellt durch die Dialysance. Bei der Bestimmung dieser Dialysance kommt der Kenntnis der Bluteingangskonzentration eines Stoffes, der am Stoffaustausch des Dialysators teilnimmt, eine besondere Bedeutung zu. Diese Bluteingangskonzentration kann gemäß der Erfindung sehr einfach und schnell dialysatseitig durch eine kurze Unterbrechung des Zuflusses von Dialysierflüssigkeit in den Dialysator und des Abflusses von Dialysierflüssigkeit aus dem Dialysator bei nicht angehaltenem Blutkreislauf bestimmt werden, wobei der im Dialysator verbleibenden Restmenge an Dialysierflüssigkeit gestattet wird, in ein diffusives Gleichgewicht mit dem Blut zu kommen, wodurch diese Restmenge die Leitfähigkeit des Blutes und damit dessen Eingangskonzentration annimmt. Eine Ultrafiltration findet während der Messung nicht statt. Nach der Einstellung des Gleichgewichtszustandes wird wieder in den Normalbetrieb geschaltet und die Konzentration nach einer gewissen zeitlichen Verzögerung am dialysatseitigen Messfühler in der Ableitung gemessen. Durch anschließende übliche Messungen des Flusses der Dialysierflüssigkeit und der Konzentrationen stromauf und stromab des Dialysators lässt sich dann die Dialysance D bestimmen.

EP 0 407 737 A1 offenbart ein Verfahren zur Prüfung einer Hämodialysemembran eines Dialysators, welcher eine Blutkammer und eine Dialysierflüssigkeitskammer aufweist. Um sicher zu stellen, dass in der Membran keine Fehlstellen oder Lecks vorhanden sind, welche von der Richtung des Druckgradienten zwischen der Dialysierflüssigkeitskammer und der Blutkammer abhängig sind, wird erfindungsgemäß in einem Prüfschritt ein Überdruck in der mit Luft befüllten Blutkammer aufgebracht, während in einem zweiten Prüfschritt die Blutkammer des Dialysators mit einem Unterdruck beaufschlagt wird.

## Kurzbeschreibung der Erfindung

Der vorliegenden Erfindung liegt u.a. die Aufgabe zugrunde, die Dialysierflüssigkeit in ihrer Zusammensetzung automatisch so anzupassen, dass die Plasma-Natrium-Konzentration beibehalten oder dem Patienten eine definierte Menge Natrium hinzugefügt oder entzogen wird.

Diese Aufgabe wird gelöst durch eine Vorrichtung nach Patentanspruch 1.

Dementsprechend wird also eine mit der Plasma-Natrium-Konzentration des Blutes korrelierte Größe gemessen und die Zusammensetzung der (frischen) Dialysierflüssigkeit in Abhängigkeit der gemessenen Größe dergestalt eingestellt, dass die Plasma-Natrium-Konzentration des Blutes zumindest am Ende der Blutbehandlung den gleichen Wert aufweist wie zu Beginn. Die Messung erfolgt dadurch, dass zu Beginn der Dialysetherapie die Dialysevorrichtung kurz in einen Bypass-Modus geschaltet wird, um im Anschluss auf der Seite der verbrauchten Dialysierflüssigkeit einen Wert zu ermitteln, der stark mit der Plasma-Natrium-Konzentration des Patienten korreliert. Dieser Wert wird ggf. korrigiert. Die Dialysevorrichtung setzt die (frische) Dialysierflüssigkeit daraufhin automatisch so zusammen, dass die Plasma-Natrium-Konzentration entsprechend des ermittelten und ggf. korrigierten Werts gesenkt wird, erhöht wird oder aber konstant bleibt.

In anderen Worten wird eine Vorrichtung und ein zugehöriges, nicht in den Schutzbereich der Ansprüche fallendes Verfahren, zur extrakorporalen Blutbehandlung, insbesondere zur Hämodialyse, bereitgestellt mit einem Dialysator, dem frische Dialysierflüssigkeit über eine Dialysierflüssigkeits-Zuführleitung zuführbar ist und von dem verbrauchte Dialysierflüssigkeit über eine Dialysierflüssigkeits-Abführleitung abführbar ist, einer Bypass-Leitung, welche die Dialysierflüssigkeits-Zuführ- und Abführleitungen unter Umgehung des Dialysators verbindet und wahlweise für ein Kurzschließen des Dialysators freigebbar ist, einer ersten Messeinrichtung zur Messung einer mit der Plasma-Natrium-Konzentration eines im Dialysator zu reinigenden Blutes korrelierten Größe, die dem Dialysator unmittelbar nachgeschaltet ist, und einer Proportionierungseinheit zur automatischen Einstellung der Zusammensetzung der Dialysierflüssigkeit in Abhängigkeit der gemessenen Größe dergestalt, dass die Plasma-Natrium-Konzentration des Blutes zumindest am Ende der Blutbehandlung den gleichen Wert aufweist, wie zu Beginn. Ferner hat die Vorrichtung eine Steuereinheit zur Ausgabe von Befehlen und zur Erfassung zumindest der Messwerte bzw. Zustände der Messeinrichtung und der Proportionierungseinheit sowie eine Toxin-Messeinrichtung zur Messung einer Toxinbelastung der vom Dialysator abgegebenen verbrauchten Dialysierflüssigkeit, wobei die gemessene Toxinbelastung zur Korrektur einer Leitfähigkeitsmessung der ersten Messeinrichtung verwendet wird / verwendbar ist. Dabei sind die zwischen der Bypass-Leitung und dem Dialysator sich befindenden Leitungsabschnitte der Dialysierflüssigkeits-Zuführ- und -Abführleitungen pumpenfrei gehalten, sodass die korrelierte Größe zumindest zu Beginn der extrakorporalen Blutbehandlung mittels einer zwischengeschalteten Bypass-Betriebsart bei Stillstand der Dialysierflüssigkeit in dem Dialysator ermittelt wird. D.h., die die Steuereinheit ist dazu ausgelegt, dass zumindest zu Beginn der extrakorporalen Blutbehandlung Dialysierflüssigkeit mittels einer zwischengeschalteten Bypass-Betriebsart bei Stillstand in dem Dialysator verbleibt, sodass nach Beendigung der Bypass-Betriebsart die Messvorrichtung dazu ausgelegt ist, die korrelierte Größe zu ermitteln.

Durch die vorgeschlagene Lösung kann ein Dialysevorgang bereitgestellt werden, durch den hohe Ultrafiltrationsraten mittel- und langfristig vermieden werden können und die Wasser- und Elektrolytmenge im Blut nur moderat geändert werden. Ferner ist nur ein geringer bis gar kein apparativer Aufwand erforderlich, da die hier vorgeschlagenen Messvorrichtungen in vielen herkömmlichen Dialysevorrichtungen bereits verfügbar sind.

Spezifische vorteilhafte Ausführungsformen der vorliegenden Erfindung sind in den Unteransprüchen angegeben.

**Kurzbeschreibung der Figuren**

Im Folgenden werden bevorzugte Ausführungsbeispiele der vorliegenden Erfindung unter Bezugnahme auf die beiliegenden Zeichnungsfiguren näher beschrieben. Es zeigen:

Fig. 1 ein schematisches Blockschaltbild einer Dialysevorrichtung gemäß einem ersten Ausführungsbeispiel;

Fig. 2 ein Flussdiagramm eines Dialysesteuerverfahrens gemäß dem ersten Ausführungsbeispiel;

Figuren 3A und 3B Zeitdiagramme mit Leitfähigkeitsverläufen bei längerer bzw. kürzerer Bypassdauer;

Fig. 4 ein schematisches Blockschaltbild einer Dialysevorrichtung gemäß einem zweiten Ausführungsbeispiel;

Fig. 5 ein schematisches Blockschaltbild einer Dialysevorrichtung gemäß einem dritten Ausführungsbeispiel;

Fig. 6 ein schematisches Diagramm eines neuronalen Netzes für eine Dialysevorrichtung gemäß einem vierten Ausführungsbeispiel;

Fig. 7 ein Zeitdiagramm mit Konzentrations- und Leitfähigkeitsverlauf bei der Dialysevorrichtung gemäß dem vierten Ausführungsbeispiel; und

Fig. 8 ein Zeitdiagramm mit einem Extinktionsverlauf bei der Dialysevorrichtung gemäß dem vierten Ausführungsbeispiel.

Im Folgenden werden bevorzugte Ausführungsbeispiele der vorliegenden Erfindung am Beispiel einer Vorrichtung und eines nicht in den Schutzbereich der Ansprüche fallenden Verfahrens zur Durchführung einer isonaträmischen Dialyse beschrieben.

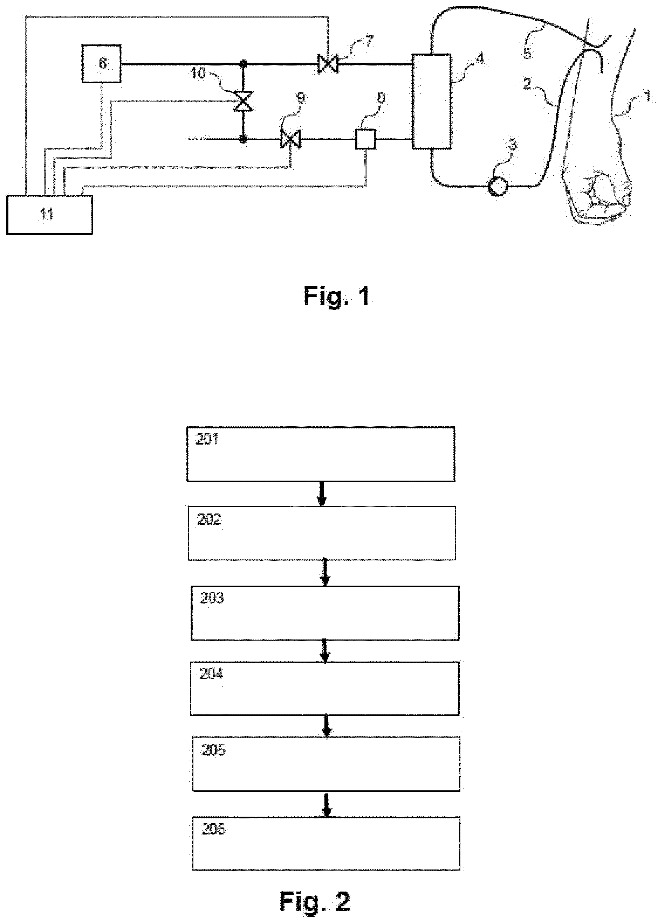

Fig. 1 zeigt ein schematisches Blockschaltbild einer Dialysevorrichtung gemäß einem ersten Ausführungsbeispiel.

Einem Dialysepatienten 1 wird hier über ein arterielles Schlauchsystem 2 mithilfe einer Fördereinrichtung (Pumpe) 3 Blut entzogen. Das Blut gelangt in einen Dialysator 4, wo es von Toxinen und überschüssigem Wasser mittels Diffusion, Konvektion und/oder Ultrafiltration befreit wird. Anschließend wird das verarbeitete Blut dem Patienten 1 über ein venöses Schlauchsystem 5 wieder zurückgeführt. Die Entnahme und Rückgabe kann auch über eine gemeinsame Kanüle erfolgen.

Bei dem Dialysator 4 kann es sich bspw. um einen handelsüblichen Dialysator handeln, wie er für extrakorporale Blutbehandlungen eingesetzt wird. Er beinhaltet mehrere Hohlfaserkapillare, die durch eine semipermeable Membran charakterisiert sind. Das Patientenblut durchströmt die Kapillare. Im Inneren des Dialysators 4 werden sie von außen von der frischen Dialysierflüssigkeit umspült, welche Toxine und weitere Schadstoffe aus dem Blut aufnimmt. Die frische Dialysierflüssigkeit wird in einer Proportionierungseinheit 6 hergestellt. Wie bereits eingangs erwähnt, können hierfür verschiedene bekannte Prinzipien verwendet werden. Beispiele sind die leitfähigkeitsgesteuerte Proportionierung, die volumetrische Proportionierung oder eine Mischform aus beiden. Üblicherweise werden hierzu hochreines Wasser, eine basische Komponente und eine saure Komponente miteinander vermischt.

In einem Hauptschluss strömt die frische Dialysierflüssigkeit durch ein erstes steuerbares Ventil 7 in den Dialysator 4, nimmt dort die Schadstoffe aus dem Blut auf, und gibt ggf. andere Stoffe, insbesondere Hydrogencarbonat und/oder andere Elektrolyte, an das Blut ab. Nach Durchlaufen des Dialysators 4 wird die Dialysierflüssigkeit als verbrauchte Dialysierflüssigkeit bezeichnet.

Gemäß den nachfolgenden Ausführungsbeispielen passiert die verbrauchte Dialysierflüssigkeit eine Messvorrichtung 8 sowie ein zweites steuerbares Ventil 9. Eine Bilanzierungseinheit (nicht gezeigt), stellt bspw. durch Vergleich und ggf. Anpassung des Flusses der frischen und der verbrauchten Dialysierflüssigkeit sicher, dass dem Patienten genau die vorgeschriebene Menge an überschüssigem Wasser entzogen wird. Im Hauptschluss ist ferner ein drittes steuerbares Ventil 10 geschlossen. Eine Steuereinheit 11 erfasst zumindest die Messwerte bzw. Zustände der Messvorrichtung 8, der Proportionierungseinheit 6 sowie der ersten bis dritten Ventile 7, 9 und 10. Des Weiteren gibt die Steuereinheit 11 Befehle an die Proportionierungseinheit 6 und an die ersten bis dritten Ventile 7, 9 und 10 aus.

Nachfolgend wird die Funktion der in Fig. 1 gezeigten Dialysevorrichtung unter Bezugnahme auf Fig. 2 näher erläutert.

Fig. 2 zeigt ein Flussdiagramm eines mittels der Dialysevorrichtung ausgeführten Dialysesteuerverfahrens gemäß dem ersten Ausführungsbeispiel.

Im Schritt 201 zu Beginn einer Dialysebehandlung, vorzugsweise innerhalb der ersten 20min, noch bevorzugter innerhalb der ersten 15min, noch mehr bevorzugt innerhalb der ersten 10min, wird der Dialysator 4 in den Bypass geschaltet. Vorzugsweise entspricht der Blutfluss der Fördereinrichtung 3 dabei einem vorgeschriebenen Wert, der höher ausfällt als der Blutfluss beim Anlegen des Patienten.

Während des Bypasses wird die frische Dialysierflüssigkeit aus der Proportionierungseinheit 6 über das im Schritt 202 durch die Steuereinheit 11 geöffnete dritte Ventil 10 am Dialysator 4 vorbei in einen Abfluss geleitet.

Das erste und zweite Ventil 7, 9 sind während des Bypasses geschlossen. Das Blut des Patienten 1 strömt dagegen weiterhin durch den Dialysator 4. Begrenzt durch das erste und zweite Ventil 7, 9 verbleibt ein Restvolumen an verbrauchter Dialysierflüssigkeit auf der Ausgangsseite des Dialysators. Streng genommen befindet sich das Restvolumen an verbrauchter Dialyseflüssigkeit im gesamten Abschnitt zwischen den Ventilen 7 und 9 einschließlich der Dialysierflüssigkeitsseite des (innerhalb des) Dialysators 4. Abhängig vom Blutfluss der Fördereinheit 3 und der Größe des Dialysators 4 wird innerhalb weniger Minuten das gesamte oder zumindest ein Teil des Restvolumens an verbrauchter Dialysierflüssigkeit auf der Seite der verbrauchten Dialysierflüssigkeit im Dialysator 4 soweit gesättigt, dass es die Konzentration der blutseitig gelösten Substanzen vollständig oder zumindest zum Teil annimmt. Bei den betrachteten blutseitig gelösten Substanzen handelt es sich um Stoffe, die klein genug sind, um die semipermeable Membran des Dialysators 4 zu passieren. Dazu zählen insbesondere freie, ungebundene Natrium-Ionen und weitere Elektrolyte sowie harnpflichtige Substanzen wie Harnstoff, Harnsäure oder Kreatinin.

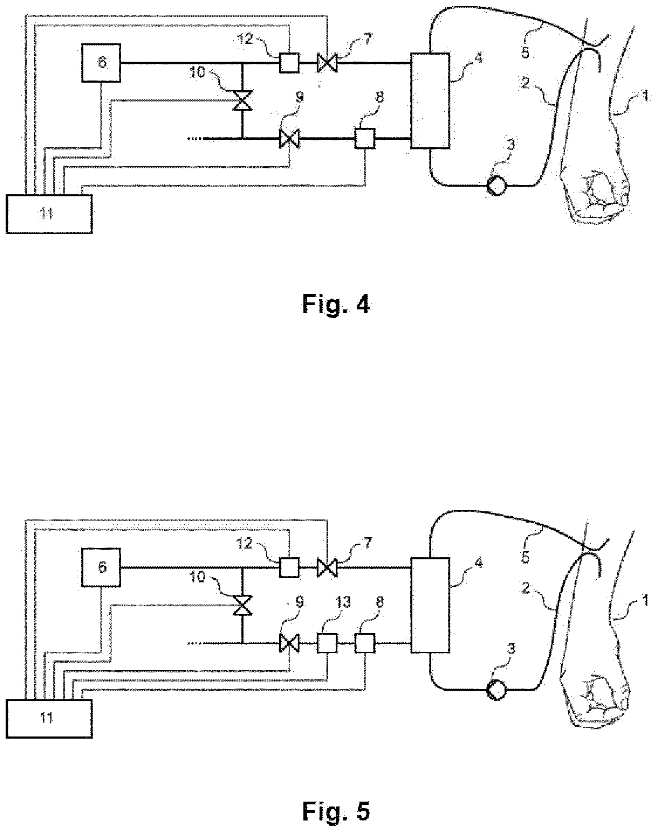

Nach Beendigung des Bypasses wird im Schritt 203 die Ventilstellung der ersten, zweiten und dritten Ventile 7, 9, 10 umgeschaltet, sodass die (frische) Dialysierflüssigkeit aus der Proportionierungseinheit 6 das zumindest teilweise angesättigte Restvolumen der verbrauchten Dialysierflüssigkeit aus dem Dialysator 4 verdrängt. Dabei passiert das Restvolumen die Messvorrichtung 8, wo es zu einer kurzzeitigen Signaländerung kommt. An dieser Stelle sei darauf hingewiesen, dass die Messvorrichtung 8 auch so positioniert sein kann, dass sie während des Bypasses weiterhin von frischer Dialysierflüssigkeit durchspült wird (also stromab zur das Ventil 10 beinnhaltenden Bypassleitung) und nicht, wie in Fig. 1 zu sehen ist, zwischen dem Dialysatorausgang und der Bypassleitung. (Gleiches gilt auch für die Messvorrichtung 13 gemäß der nachfolgend beschriebenen Fig. 5. Auch kann sich die Messvorrichtung 12 gemäß der nachfolgend beschriebenen Fig. 4, 5 sich in Flussrichtung vor (stromauf) der Bypassleiung (auf Seiten der verbrauchten Dialysierflüssigkeit) befinden).

Der Wert im Extremum (Minimum oder Maximum) der Signaländerung korreliert dabei stark mit demjenigen Wert, der am Bluteingang des Dialysators 4 vorliegt.

Vorzugsweise handelt es sich bei der Messvorrichtung 8 um eine temperaturkompensierende Leitfähigkeitszelle. Die Leitfähigkeit im Extremum nach Bypassende entspricht in dem Fall der Leitfähigkeit des Plasmawassers bzw. des Plasmas des Patienten 1.

Alternativ kann als Messvorrichtung 8 auch eine stoffspezifische Messvorrichtung, wie z.B. eine ionenselektive Elektrode, oder eine optische Messvorrichtung eingesetzt werden, sodass der blutseitige Wert nichtinvasiv und direkt ermittelt werden kann.

Das vorstehend beschriebene Bypassverfahren beruht in seinen Grundzügen auf dem Patent DE 197 34 992 C1, welches auf die Bestimmung der Dialysierfähigkeit (Dialysance) abzielt.

Gemäß den vorliegenden Ausführungsbeispielen wird dieses Verfahren nun dergestalt weiterentwickelt, dass die Dialysierflüssigkeit in ihrer Zusammensetzung mithilfe der Proportionierungseinheit 6 so angepasst wird, dass dem Patienten 1 in dem Maße Natrium entzogen wird, dass die Plasma-Natrium-Konzentration gegen Ende der Therapie der anfänglichen Konzentration entspricht (isonaträmische Dialyse). Hierfür wird der Messwert des lokalen Signalextremums im Schritt 204 in Fig. 2 ausgewertet und im Schritt 205 über die Steuereinheit 11 an die Proportionierungseinheit 6 weitergeleitet. Letztere mischt die Dialysierflüssigkeit nun im Schritt 206 so an, dass die Plasma-Natrium-Konzentration im weiteren Verlauf der Dialyse nicht verändert wird oder zumindest gegen Ende den gleichen Wert aufweist wie zu Beginn. Um mögliche Änderungen nachverfolgen zu können, wird der Bypass mit den vorgenannten Verfahrensschritten 201 bis 206 in regelmäßigen Abständen wiederholt.

Bei dem vorstehend beschriebenen Bypassverfahren muss jedoch während des Bypasses ein diffusives Gleichgewicht zwischen der Blutseite und der Seite der verbrauchten Dialysierflüssigkeit erreicht werden. Für niedrige Blutflüsse und große Dialysatoren kann dies allerdings mehrere Minuten dauern. Hervorzuheben ist dabei, dass das Blut aufgrund des stehenden Restvolumens auf der Seite der verbrauchten Dialysierflüssigkeit nicht effizient genug gereinigt wird. Bei Durchführung eines einzelnen Bypasses ist dies zwar zu vernachlässigen. Sollen jedoch mehrere Bypässe im Laufe der Dialysebehandlung durchgeführt werden, müsste die Behandlung verlängert werden, um eine ausreichend hohe Dialysedosis erreichen zu können.

Gemäß den Ausführungsbeispielen kann daher vorgesehen sein, die Bypasszeit zu verkürzen, indem auf ein diffusives Gleichgewicht zwischen der Blutseite und der Seite der verbrauchten Dialysierflüssigkeit verzichtet wird. Labormessungen haben ergeben, dass bereits 14s Bypasszeit ausreichend sind, um nach Bypassende eine Signaländerung zu erzeugen, die 50% einer vollständigen Sättigung entspricht.

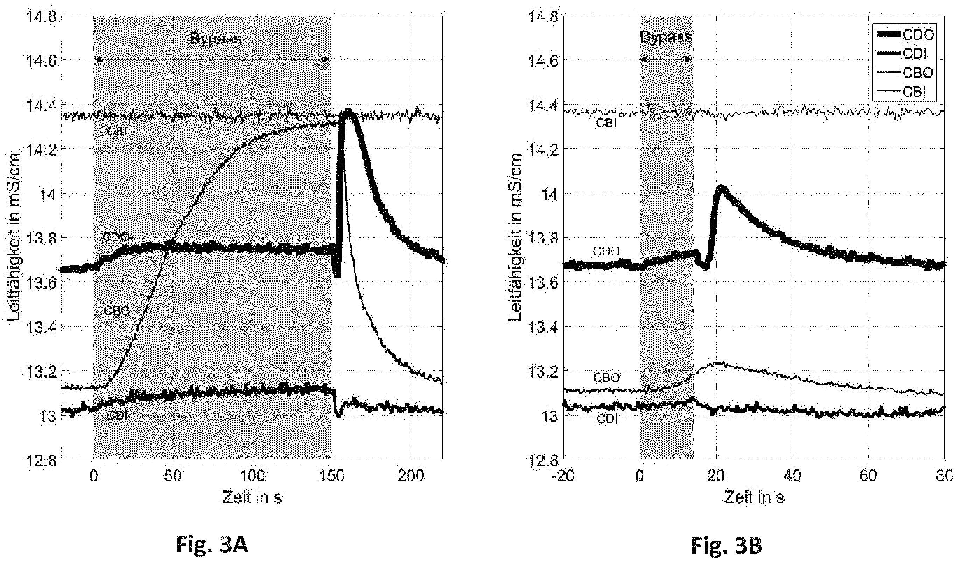

Die Figuren 3A und 3B zeigen Zeitdiagramme mit Leitfähigkeitsverläufen CBI am Bluteingang (des Dialysators), CBO am Blutausgang (des Dialysators), CDI am Dialysierflüssigkeitseingang (des Dialysators) und CDO am Dialysierflüssigkeitsausgang (des Dialysators) bei längerer bzw. kürzerer Bypassdauer zur Verdeutlichung der vorgenannten Wirkung, wobei das linke Diagramm einem Bypass mit längerer Dauer und das rechte Diagramm einem Bypass mit kürzerer Dauer entspricht. Die Leitfähigkeit am Dialysierflüssigkeitsausgang (des Dialysators) beträgt direkt vor Durchführung des Bypasses etwa 13.6 mS/cm. Nach einer Bypassdauer von 2.5min kann von einer vollständigen Sättigung ausgegangen werden. Dies ist auch daran zu erkennen, dass die hier dargestellten Leitfähigkeiten am Bluteingang und Blutausgang (des Dialysators) nach 2.5min einander entsprechen und einen Wert von etwa 14.4 mS/cm aufweisen.

Die Leitfähigkeit (CDO) am Dialysierflüssigkeitsausgang (des Dialysators) beträgt im Extremum nach Bypassende ebenfalls etwa 14.4 mS/cm, was einer Änderung von 0.8 mS/cm entspricht. Im rechten Diagramm ist erkennbar, dass bei einer Bypassdauer von lediglich 14s die Leitfähigkeit am Dialysierflüssigkeitsausgang (des Dialysators) von 13.6 mS/cm um 0.4 mS/cm auf 14.0 mS/cm ansteigt. Also genau um die Hälfte von 0.8 mS/cm.

Über folgende Gleichung kann nun auf die Leitfähigkeit geschlossen werden, die am Bluteingang (des Dialysators) vorliegt:

$$CBI_{calc} = CDO_{pre} + k \cdot \left( CDO_{ext} - CDO_{pre} \right)$$

$CBI_{calc}$ kennzeichnet dabei den mit dem Bypassverfahren nichtinvasiv berechneten Wert am Bluteingang (des Dialysators), $CDO_{pre}$ den Wert am Dialysierflüssigkeitsausgang (des Dialysators) direkt vor Durchführung des Bypasses, der mit der Messvorrichtung 8 erfasst wird, und $CDO_{ext}$ den Wert im Extremum am Dialysierflüssigkeitsausgang (des Dialysators) nach Bypassende. Bei diesen Werten kann es sich auch um gefilterte Werte handeln. Sie können z.B. der Mittelwert oder Median eines definierten Zeitabschnittes sein. Der Faktor k ist für einen 14s-Bypass bei einer mittleren Dialysatorgröße und einem mittleren Blutfluss gleich 2. Falls andere Blutflüsse oder Dialysatoren oder Dialysierflüssigkeitsflüsse verwendet werden sollten, kann auch eine Justierung des Faktors k durchgeführt werden. Hierzu wird zuerst ein ausreichend langer Bypass durchgeführt, wobei von einem diffusiven Gleichgewicht zwischen Blut- und Dialysierflüssigkeitsseite ausgegangen werden kann. Zeitnah, vorzugsweise innerhalb von 1 bis 2min nach Beendigung des langen Bypasses, wird dann ein kurzer Bypass mit einer Dauer von 14s oder mit unterschiedlichen Zeiten durchgeführt. Alternativ kann die Reihenfolge auch geändert werden.

Durch Vergleich der Messwerte beider Bypässe wird anschließend der Faktor k ermittelt. Beispielsweise kann folgender Quotient aufgestellt werden:

$$k = \frac{\langle CDOext - CDOpre \rangle_{long}}{\langle CDOext - CDOpre \rangle_{short}}$$

Der Zähler ist die Differenz der entsprechenden Werte aus dem langen Bypass und der Nenner ist die Differenz der entsprechenden Werte aus dem kurzen Bypass.

Es folgt nun eine Beschreibung eines zweiten Ausführungsbeispiels.

Fig. 4 zeigt ein schematisches Blockschaltbild einer Dialysevorrichtung gemäß dem zweiten Ausführungsbeispiel.

Beim zweiten Ausführungsbeispiel ist in der Zuführleitung der frischen Dialysierflüssigkeit eine weitere Messvorrichtung 12 vorhanden, wobei die beiden Messvorrichtungen 8, 12 vom selben Typ sein können. Die Messvorrichtung 12 kann auch eine Komponente der Proportionierungseinheit 6 sein, da letztere häufig bereits eine entsprechende Messvorrichtung beinhaltet. Vorzugsweise handelt es sich auch bei der weiteren Messvorrichtung 12 um eine temperaturkompensierende Leitfähigkeitssonde. Die weitere Messvorrichtung 12 ist für die Durchführung einer isonaträmischen Dialyse nicht zwingend erforderlich. Allerdings kann sie in Kombination mit der Messvorrichtung 8 dazu verwendet werden, den Auswertealgorithmus des Signals an der Messvorrichtung 8 zu vereinfachen. Anhand des Vergleichs der Messwerte an beiden Messvorrichtungen 8, 12 ist schnell ersichtlich, ob nach Beendigung eines Bypasses der Extremwert ein Minimum oder ein Maximum ist. Ist der Wert an der Messvorrichtung 8 größer bzw. kleiner als derjenige an der weiteren Messvorrichtung 12, so wird nach Bypassende ein Maximum bzw. Minimum gesucht.

Es folgt nun eine Beschreibung eines dritten Ausführungsbeispiels.

Fig. 5 zeigt ein schematisches Blockschaltbild einer Dialysevorrichtung gemäß dem dritten Ausführungsbeispiel.

Wie bereits erwähnt, handelt es sich bei der Messvorrichtung 8 vorzugsweise um eine temperaturkompensierende Leitfähigkeitssonde. Obwohl Natrium und seine Anionen, insbesondere Chlorid und Hydrogencarbonat, solche Stoffe in Flüssigkeiten wie Dialysierflüssigkeit oder Plasmawasser darstellen, die am stärksten zur Leitfähigkeit beitragen, wird die Leitfähigkeitsmessung von weiteren Stoffen beeinflusst, sodass eine einfache Umrechnung zwischen Leitfähigkeit und Natrium-Konzentration nicht möglich ist. So können bspw. erhöhte Kalium-Werte die Leitfähigkeit erhöhen. Es gibt aber auch Stoffe, die per se nicht leitfähig sind und dennoch die Leitfähigkeit beeinträchtigen können. Es kann beispielhaft gezeigt werden, dass die Zugabe von nicht leitfähiger Glucose die Leitfähigkeit einer ansonsten leitfähigen Lösung herabsenken kann, da Glucose die Beweglichkeit der leitenden Ionen beeinträchtigt. Ähnliche Effekte werden von Toxinen und anderen harnpflichtigen Substanzen verursacht. Insbesondere zu Beginn einer Dialysebehandlung passieren viele dieser Stoffe die semipermeable Membran im Dialysator 4 und gelangen so auf die Dialysierflüssigkeitsseite. Die Messvorrichtung 8 misst deshalb eine Leitfähigkeit, die aufgrund dieser Substanzen herabgesetzt sein kann. Würde nun aber die Proportionierungseinheit 6 exakt diese Leitfähigkeit anmischen, so würde dem Patienten dadurch im Laufe der Dialysebehandlung in unerwünschter Weise Natrium entzogen. Um diesem Effekt entgegenzuwirken, ist es notwendig, die Leitfähigkeit an der Messvorrichtung 8 zu korrigieren. Eine starre Korrektur der Leitfähigkeit durch Addieren eines festen Betrags ist allerdings nachteilhaft, da die Belastung mit Toxinen von Patient zu Patient und von Behandlung zu Behandlung unterschiedlich ausfallen kann.

Erfindungsgemäß ist daher vorgesehen, am (Dialysator-) Ausgang der verbrauchten Dialysierflüssigkeit neben der Messvorrichtung 8 eine dritte Messvorrichtung 13 anzubringen, die dazu dient, die Toxinbelastung der verbrauchten Dialysierflüssigkeit zu bestimmen. Vorzugsweise handelt es sich dabei um einen optischen Sensor, der die Absorptionseigenschaft der verbrauchten Dialysierflüssigkeit misst. Vorzugsweise wird die Absorptionseigenschaft im ultravioletten Bereich zwischen 235nm und 400nm gemessen. Weiter bevorzugt wird die Absorptionseigenschaft von Licht mit einer Wellenlänge von 285±15nm gemessen. Alternativ ist auch ein enzymatischer oder ein anderer elektrochemischer Sensor denkbar.

Zur Korrektur der mithilfe des Bypassverfahrens ermittelten Leitfähigkeit kann die Absorptionseigenschaft der verbrauchten Dialysierflüssigkeit direkt vor Durchführung eines Bypasses herangezogen werden. Alternativ ist auch die Absorptionseigenschaft nach Bypassende denkbar, die zeitgleich mit dem Signalextremum an der Messvorrichtung 8 auftritt.

Eine mögliche Absorptionseigenschaft ist die Extinktion. Die Leitfähigkeit wird dabei korrigiert, indem Leitfähigkeit und Extinktion mathematisch miteinander kombiniert werden. Im einfachsten Fall kann dies bspw. auf nachstehender linearen Gleichung basieren:

$$CBIcalc, korr = a \cdot CBIcalc + b \cdot E + c$$

wobei CBIcalc,korr die basierend auf der Messung mit der Messvorrichtung 8 korrigierte berechnete Leitfähigkeit am Bluteingang kennzeichnet, a und b Faktoren sind, E die mit der dritten Messvorrichtung 13 am (Dialysator-) Ausgang der verbrauchten Dialysierflüssigkeit gemessene Extinktion darstellt, und c eine Konstante ist.

Es folgt nun eine Beschreibung eines auf einem neuronalen Netz basierenden vierten Ausführungsbeispiels.

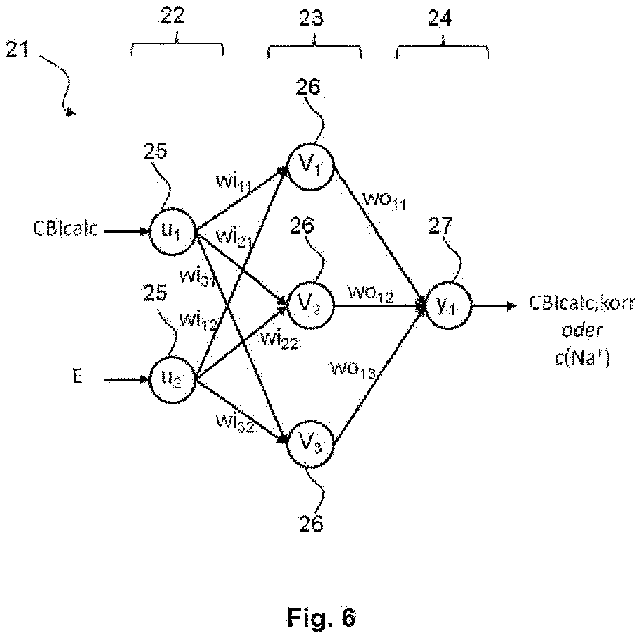

Fig. 6 zeigt ein schematisches Diagramm des neuronalen Netzes für eine Dialysevorrichtung gemäß dem vierten Ausführungsbeispiel.

Versuche haben gezeigt, dass insbesondere künstliche neuronale Netze sehr gute Ergebnisse erzielen. Künstliche neuronale Netze sind in der Lage, nahezu alle messbaren Funktionen beliebig genau zu approximieren. In der Regel bestehen sie aus einer Eingangsschicht, mindestens einer versteckten Schicht und einer Ausgangsschicht.

In Fig. 6 ist ein solches neuronales Netz 21 gezeigt. Eine Eingangsschicht 22 beinhaltet hier zwei Neuronen 25, in welchen als Eingangsgrößen die Werte für die berechnete Leitfähigkeit am Bluteingang (CBIcalc) und die Extinktion (E) normiert werden. Gewichtet mit den Wichtungen wi werden sie an Zwischenschichtneuronen 26 einer Zwischenschicht 23 übergeben, wo die gewichteten Eingänge u gemäß nachfolgender Formel aufaddiert werden:

$$\sigma_j = \sum_{k=1}^{r} wi_{jk} u_k$$

Neben diesen Eingängen können auch weitere konstante Eingänge, sogenannte Schwellwerte, zum Einsatz kommen. Schwellwerte können dabei entweder -1 oder +1 sein und können ebenfalls gewichtet werden. Außerdem ist es möglich, auch andere Messwerte anderer, z.B. auch externer Messvorrichtungen als Eingänge einzuspeisen. (Weitere Eingänge könnten z.B. die Leitfähigkeit am Dialysierflüssigkeitseingang und/oder Dialysierflüssigkeitsausgang sein. Noch weitere Eingänge könnten die Kalium- und Hydrogencarbonat-Konzentrationen im Plasma sein).

So ist bspw. auch denkbar, z.B. handelsübliche Hämatokrit-Sensoren einzubeziehen. Des Weiteren bietet es sich an, nicht nur starre Messwerte, sondern auch Messwertänderungen zu betrachten. Beispiele hierfür sind Änderungen der ermittelten Leitfähigkeiten und/oder Absorptionseigenschaften und/oder Hämatokrit-Werte und/oder Änderungen des relativen Blutvolumens und/oder der Sauerstoffsättigung des Blutes zwischen mindestens zwei Bypässen.

Die Summen der gewichteten Eingänge werden in jedem Zwischenschichtneuron anschließend an eine sigmoide Aktivierungsfunktion übergeben. Grundsätzlich sind beliebige sigmoide Funktionen denkbar. Bevorzugt kann eine Tangens-Hyperbolicus-Funktion verwendet werden, die sich dadurch auszeichnet, dass ihre Funktionswerte für beliebige Eingangswerte in einem Bereich zwischen den Werten -1 und +1 liegen. Der Tangens-Hyperbolicus-Funktion ist wie folgt definiert:

$$g(\sigma) = \frac{e^{\sigma} - e^{-\sigma}}{e^{\sigma} + e^{-\sigma}} = \frac{e^{2\sigma} - 1}{e^{2\sigma} + 1} = 1 - \frac{2}{e^{2\sigma} + 1}$$

Die Ausgangswerte V der Zwischenschichtneuronen 26 ergeben sich somit wie folgt:

$$V_j = g(\sigma_j) = g\left(\sum_{k=1}^{r} wi_{jk} u_k\right)$$

Schließlich werden Ausgangswerte mit Ausgangsgewichtungen wo versehen und an ein Ausgangsneuron 27 einer Ausgangsschicht 24 weitergeleitet, wo eine Summe gebildet wird. Auch hier kann ein gewichteter Schwellwert hinzuaddiert werden.

Somit ergibt sich als Ausgang $y_1$ für das Ausgangsneuron 27:

$$y_1 = \left( \sum_{j=1}^{s} wo_{1j} V_j \right) + bo_1$$

wobei $bo_1$ den gewichteten Schwellwert des Ausgangsneurons 27 darstellt.

Es sei darauf hingewiesen, dass die Schwellwerte in Fig. 6 nicht dargestellt sind.

Der Netzausgang ist die korrigierte berechnete Leitfähigkeit am Bluteingang, die mit dem Bypassverfahren nichtinvasiv am (Dialysator-)Ausgang der verbrauchten Dialysierflüssigkeit mittels der Messvorrichtung 8 bestimmt wurde. Selbstverständlich ist es auch möglich, direkt eine Natrium-Konzentration $c(Na^+)$ ausgeben zu lassen, die am Bluteingang vorliegt. Alternativ können auch Dosierförderraten für die Proportionierungseinheit 6 oder Mischungsverhältnisse ermittelt werden.

Die Werte für $CBI_{calc,korr}$ bzw. $c(Na^+)$, also die Leitfähigkeit bzw. Natrium-Konzentration, liegen nun zur Durchführung einer isonaträmischen Dialyse auch für die Dialysierflüssigkeit vor und können von der Proportionierungseinheit 6 angemischt werden.

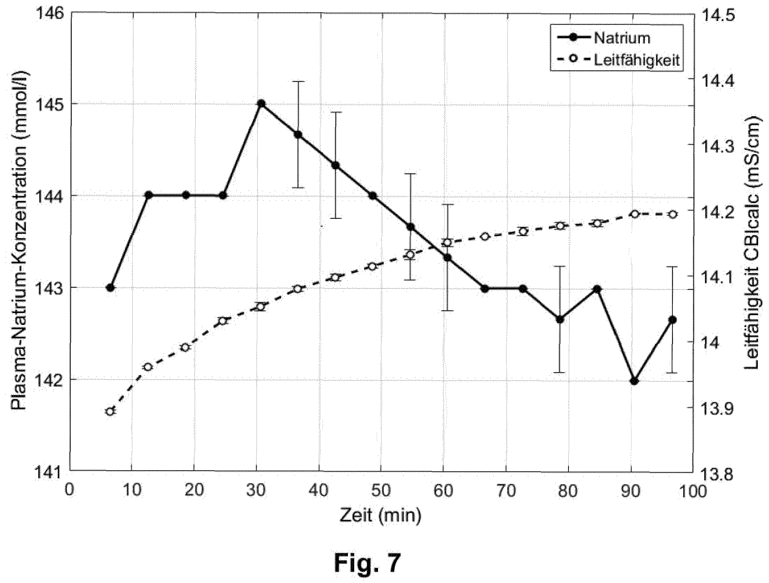

Fig. 7 zeigt ein Zeitdiagramm mit Konzentrations- und Leitfähigkeitsverlauf bei der Dialysevorrichtung gemäß dem vierten Ausführungsbeispiel als Ergebnis einer auf vorgenannte Weise durchgeführten Dialyse. Hierzu wurden 6l frisches Blut dialysiert. Die Plasma-Natrium-Konzentration betrug zu Beginn der Dialyse 143mmol/l. Im Verlauf der Behandlung steigt die Konzentration leicht an, fällt aber dann allmählich auf $142.7 \pm 0.6$mmol/l ab. Die über das Bypassverfahren berechnete Leitfähigkeit $CBI_{calc}$ betrug zu Beginn etwa 13.9 mS/cm. Dieser Wert und die dazugehörige erste Extinktion wurden dem neuronalen Netz eingegeben. Das Netz berechnete dann als korrigierte Leitfähigkeit einen Wert von 14.2 mS/cm. Dieser wurde an die Steuereinheit 11 übergeben, was dazu führte, dass die Proportionierungseinheit 6 die Dialysierflüssigkeit so zusammenmischte, dass in dieser eine Natrium-Konzentration von 143 mmol/l erhalten wurde. Übertragen auf die Gesamtleitfähigkeit der Dialysierflüssigkeit entsprechen 143 mmol/l in etwa 14.2 mS/cm.

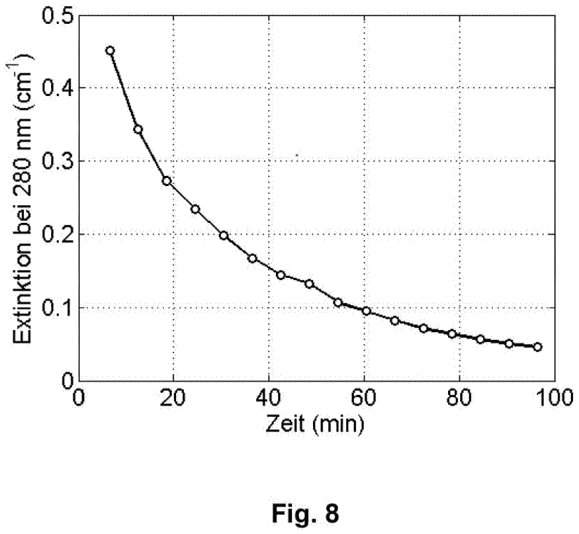

Fig. 8 zeigt ein Zeitdiagramm mit dem zugehörigen Extinktionsverlauf bei der Dialysevorrichtung gemäß dem vierten Ausführungsbeispiel. Die Emissionswellenlänge des optischen Sensors der dritten Messvorrichtung 13 betrug hier 280 nm.

Selbstverständlich ist es auch möglich, nach anfänglicher Ermittlung der Plasma-Natrium-Konzentration die frische Dialysierflüssigkeit so einzustellen, dass dem Patienten 1 eine definierte Menge Natrium entzogen oder verabreicht wird. Darüber hinaus ermöglicht das vorgeschlagene Verfahren die Bestimmung der absolut entzogenen Menge an Natrium. Bei bekanntem Ultrafiltrationsvolumen und der Kenntnis der Plasma-Natrium-Konzentration im Verlauf der Dialysebehandlung entspricht die entzogene Menge an Natrium dem Produkt aus Ultrafiltrationsvolumen und Plasma-Natrium-Konzentration.

Um das Verfahren für das medizinische Personal und/oder den Anwender transparent zu gestalten, kann vorgesehen sein, Daten der Messvorrichtungen und/oder Charakteristika der von der Proportionierungseinheit 6 angemischten Dialysierflüssigkeit auf einem Bildschirm oder einem Datenmanagementsystem anzeigen zu lassen. Das betrifft insbesondere Leitfähigkeiten, Konzentrationen, Extinktionen, pH-Werte, Temperaturen und Drücke.

Es kann außerdem vorgesehen sein, Daten nur als Empfehlung zur Verfügung zu stellen. So kann das medizinische Personal basierend auf diesen empfohlenen Daten selbst entscheiden, ob der Empfehlung nachgegangen werden soll oder nicht. Eine hypo-, iso- oder hypernaträmische Dialyse muss demnach nicht automatisch stattfinden.

Es kann vorgesehen sein, die Gewichtungen des neuronalen Netzes oder die Faktoren a und b und die Konstante c werksseitig zu ermitteln und einzustellen. Allerdings ist es auch möglich, diese Werte einstellbar bzw. lernbar zu gestalten. Beispielhaft sei hier auf die Plasma-Natrium-Konzentration hingewiesen, die z.B. bei einer routinemäßigen Laboruntersuchung des Patientenblutes kurz vor Beginn der Dialysebehandlung ermittelt werden kann. Das medizinische Personal und/oder der Anwender kann diesen gemessenen Wert direkt an der Dialysevorrichtung oder in ein Datenmanagementsystem eingeben. Dieser Wert wird dann mit dem berechneten Wert verglichen. Der berechnete Wert kann dann ggf. durch den gemessenen Wert ersetzt werden. Gleichzeitig können die oben genannten Wichtungen und Faktoren von der Dialysevorrichtung oder dem Datenmanagementsystem neu ermittelt und angepasst werden.

Ferner kann vorgesehen sein, erhobene Daten auf einer Patientenkarte, in der Dialysevorrichtung und/oder in einem Datenmanagementsystem abzuspeichern. In den vergangenen Jahren durchgeführte Studien haben ergeben, dass die Plasma-Natrium-Konzentration eines Patienten im Vergleich zu anderen Parametern relativ konstant ist (sogenannte Setpoint-Theorie).

Die gespeicherten Daten, insbesondere die berechnete Leitfähigkeit $CBI_{calc}$ und die korrigierte, berechnete Leitfähigkeit $CBI_{calc,korr}$ sowie die daraus ermittelte Plasma-Natrium-Konzentration $c(Na^+)$ können mit Mitteln der deskriptiven Statistik (z.B. Mittelwert, Standardabweichung, zeitliche Korrelationen etc.) ausgewertet werden, um eventuelle Veränderungen zu erkennen, was auf eine allgemeine Änderung des gesundheitlichen Zustands hindeuten könnte.

Zusammenfassend wurden eine Vorrichtung und ein Verfahren zur extrakorporalen Blutbehandlung beschrieben, wobei Blut eines Patienten von einer Dialysierflüssigkeit in einem Dialysator umspült wird und wobei eine mit der Plasma-Natrium-Konzentration des Blutes korrelierten Größe gemessen wird. Die Zusammensetzung der Dialysierflüssigkeit wird dann in Abhängigkeit der gemessenen Größe dergestalt eingestellt, dass die Plasma-Natrium-Konzentration des Blutes zumindest am Ende der Blutbehandlung den gleichen Wert aufweist, wie zu Beginn. Zur Messung der mit der Plasma-Natrium-Konzentration des Blutes korrelierten Größe kann bspw. ein Bypass-Betrieb eingerichtet werden, bei dem die Dialysierflüssigkeit am Dialysator vorbeigeleitet wird, sodass ein Restvolumen auf der Seite der verbrauchten Dialysierflüssigkeit die Konzentration der blutseitig gelösten Subtanzen zumindest teilweise annimmt.

**Patentansprüche**

1.  Vorrichtung zur extrakorporalen Blutbehandlung, insbesondere zur Hämodialyse, mit:

    - einem Dialysator (4), dem frische Dialysierflüssigkeit über eine Dialysierflüssigkeits-Zuführleitung zuführbar ist und von dem verbrauchte Dialysierflüssigkeit über eine Dialysierflüssigkeits-Abführleitung abführbar ist;
    - einer Bypass-Leitung, welche die Dialysierflüssigkeits-Zuführ- und Abführleitungen unter Umgehung des Dialysators (4) verbindet und wahlweise für ein Kurzschließen des Dialysators (4) freigebbar ist;
    - einer ersten Messeinrichtung (8) zur Messung einer mit der Plasma-Natrium-Konzentration eines im Dialysator (4) zu reinigenden Blutes korrelierten Größe, die dem Dialysator (4) unmittelbar nachgeschaltet ist;
    - einer Proportionierungseinheit (6) zur automatischen Einstellung der Zusammensetzung der Dialysierflüssigkeit in Abhängigkeit der gemessenen Größe dergestalt, dass die Plasma-Natrium-Konzentration des Blutes zumindest am Ende der Blutbehandlung den gleichen Wert aufweist, wie zu Beginn, und
    - einer Steuereinheit (11) zur Ausgabe von Befehlen und zur Erfassung zumindest der Messwerte bzw. Zustände der Messeinrichtung (8), der Proportionierungseinheit (6), und ersten bis dritten Ventile (7, 9, 10),
    - einer Toxin-Messeinrichtung (13) zur Messung einer Toxinbelastung der vom Dialysator (4) abgegebenen verbrauchten Dialysierflüssigkeit, wobei die gemessene Toxinbelastung zur Korrektur einer Leitfähigkeitsmessung der ersten Messeinrichtung (8) verwendbar ist, **wobei**
    - die zwischen der Bypass-Leitung und dem Dialysator (4) sich befindenden Leitungsabschnitte der Dialysierflüssigkeits-Zuführ- und -Abführleitungen pumpenfrei gehalten sind und die Steuereinheit (11) dazu ausgelegt ist, dass zumindest zu Beginn der extrakorporalen Blutbehandlung Dialysierflüssigkeit mittels einer zwischengeschalteten Bypass-Betriebsart bei Stillstand in dem Dialysator (4) verbleibt, sodass nach Beendigung der Bypass-Betriebsart die Messvorrichtung (8) dazu ausgelegt ist, die korrelierte Größe zu ermitteln.

2.  Vorrichtung nach Anspruch 1, wobei die Proportionierungseinheit (6) so ausgestaltet ist, dass sie die Zusammensetzung der Dialysierflüssigkeit in Abhängigkeit der gemessenen Größe so einstellt, dass die Plasma-Natrium-Konzentration des Blutes während der Blutbehandlung gesenkt, erhöht oder im Wesentlichen konstant gehalten wird.

3.  Vorrichtung nach Anspruch 1 oder 2, wobei die erste Messeinrichtung (8) eine Leitfähigkeitsmesseinrichtung, eine stoffspezifische Messeinrichtung und/oder eine optische Messeinrichtung umfasst.

4.  Vorrichtung nach Anspruch 3, wobei die erste Messeinrichtung (8) eine temperaturkompensierende Leitfähigkeitszelle umfasst.

5.  Vorrichtung nach einem der vorhergehenden Ansprüche, wobei die Vorrichtung ausgestaltet ist, einen Bypass-Betrieb einzurichten, während dem die Dialysierflüssigkeit am Dialysator (4) vorbeigeleitet wird, sodass ein Restvolumen auf der Seite der verbrauchten Dialysierflüssigkeit die Konzentration der blutseitig gelösten Subtanzen zumindest teilweise annimmt, und das Restvolumen auf der Seite der verbrauchten Dialysierflüssigkeit zur Messung der mit der Plasma-Natrium-Konzentration des Blutes korrelierten Größe der ersten Messeinrichtung (8) zuzuführen.

6.  Vorrichtung nach Anspruch 5, wobei der Bypass-Betrieb innerhalb der ersten 20min, vorzugsweise innerhalb der ersten 15min, noch bevorzugter innerhalb der ersten 10min, der Blutbehandlung eingerichtet wird.

7.  Vorrichtung nach einem der vorgenannten Ansprüche, wobei die Proportionierungseinheit (6) die Zusammensetzung der Dialysierflüssigkeit so einstellt, dass dem Blut in dem Maße Natrium entzogen wird, dass die Plasma-Natrium-Konzentration gegen Ende der Blutbehandlung der anfänglichen Plasma-Natrium-Konzentration entspricht.

8.  Vorrichtung nach Anspruch 5 oder 6, wobei die erste Messvorrichtung (8) ausgestaltet ist, die Leitfähigkeit des Restvolumens auf der Seite der verbrauchten Dialysierflüssigkeit bei längerer und kürzerer Dauer des Bypass-Betriebs zu messen, und wobei die Vorrichtung ausgestaltet ist, durch Vergleich der erhaltenen Messergebnisse einen Faktor zu ermitteln, mittels dem die Leitfähigkeit des Plasmas bzw. Plasmawassers am Eingang des Dialysators (4) basierend auf gemessenen Leitfähigkeiten der verbrauchten Dialysierflüssigkeit vor und nach der Bypass-Betriebsart berechnet wird.

**9.** Vorrichtung nach einem der vorgenannten Ansprüche, des Weiteren umfassend eine zweite Messeinrichtung (12) zur Durchführung einer zweiten Messung der mit der Plasma-Natrium-Konzentration des Blutes korrelierten Größe in einer Zuführleitung der Dialysierflüssigkeit, wobei der Messwert der zweiten Messeinrichtung mit dem Messwert der ersten Messeinrichtung (8) verglichen wird, um festzustellen, ob ein gemessener Extremwert einem Maximum oder einem Minimum entspricht.

**10.** Vorrichtung nach Anspruch 1, wobei die Toxin-Messeinrichtung (13) einen optischen Sensor zur Messung einer Absorptionseigenschaft, insbesondere der Extinktion, der verbrauchten Dialysierflüssigkeit umfasst.

**11.** Vorrichtung nach Anspruch 10, des Weiteren umfassend ein neuronales Netz (21) zur Korrektur der Leitfähigkeitsmessung der ersten Messeinrichtung (8) unter Verwendung einer sigmoiden Aktivierungsfunktion, insbesondere einer Tangens-Hyperbolicus-Funktion, in einer Zwischenschicht.

**Claims**

**1.** An apparatus for extracorporeal blood treatment, in particular for hemodialysis, comprising:

- a dialyser (4) to which fresh dialysate can be supplied via a supply line of the dialysate, and from which used dialysate can be discharged via a discharge line of the dialysate;
- a bypass line which connects the supply line and the discharge line of the dialysate, thereby bypassing the dialyser (4), and which can selectively be opened for short circuiting the dialyser (4);
- a first measuring device (8) for measuring a variable correlated with the plasma sodium concentration of the blood to be cleaned in the dialyser (4), the first measuring device (8) being provided directly downstream of the dialyser (4);
- a proportioning unit (6) for automatically adjusting the composition of the dialysate in response to the variable measured such that the plasma sodium concentration of the blood at least at the end of the blood treatment has the same value as at the beginning, and
- a control unit (11) for issuing commands and for detecting at least the measured values or conditions of the measuring device (8), of the proportioning unit (6), and of first to third valves (7, 9, 10),
- a toxin measuring device (13) for measuring a toxin contamination of the used dialysate output by the dialyser (4), wherein the measured toxin contamination is usable for correcting a conductivity measurement of the first measuring device (8), **wherein**
- line sections of the supply line and the discharge line of the dialysate located between the bypass line and the dialyser (4) are pumpless, and the control unit (11) is adapted such that, at least at a beginning of the extracorporeal blood treatment, dialysate remains in the dialyser (4) via an interposed bypass operation during standstill, such that after the bypass mode is terminated, the measuring device (8) is adapted to determine the correlated variable.

**2.** The apparatus according to claim 1, wherein the proportioning unit (6) is configured so that it adjusts the composition of the dialysate in response to the variable measured so that during the blood treatment the plasma sodium concentration of the blood is reduced, increased or maintained substantially constant.

**3.** The apparatus according to claim 1 or 2, wherein the first measuring device (8) comprises a conductivity measuring device, a substance-specific measuring device and/or an optical measuring device.

**4.** The apparatus according to claim 3, wherein the first measuring device (8) comprises a temperature-compensating conductivity cell.

**5.** The apparatus according to one of the preceding claims, wherein the apparatus is configured to implement a bypass operation during which the dialysate is guided past the dialyser (4) so that a residual volume on the side of the used dialysate at least partially adopts the concentration of the substances dissolved on the blood side, and to supply the residual volume on the side of the used dialysate to the first measuring device (8) for measuring the variable correlated with the plasma sodium concentration of the blood.

6. The apparatus according to claim 5, wherein the bypass operation is implemented within the first 20min, preferably within the first 15min, even more preferred within the first 10min, of the blood treatment.

7. The apparatus according to one of the preceding claims, wherein the proportioning unit (6) adjusts the composition of the dialysate so that sodium is withdrawn from the blood to such an extent that the plasma sodium concentration toward the end of the blood treatment corresponds to the initial plasma sodium concentration.

8. The apparatus according to claim 5 or 6, wherein the first measuring device (8) is configured to measure the conductivity of the residual volume on the side of the used dialysate with longer and shorter durations of the bypass operation, and wherein the apparatus is configured to identify, by comparison of the obtained measuring results, a factor by means of which the conductivity of the plasma or plasma water at the inlet of the dialyser (4) is calculated on the basis of measured conductivities of the used dialysate before and after the bypass operation.

9. The apparatus according to one of the preceding claims, moreover comprising a second measuring device (12) for carrying out a second measurement of the variable correlated with the plasma sodium concentration of the blood in a supply line of the dialysate, wherein the measuring value of the second measuring device is compared to the measuring value of the first measuring device (8) to determine whether a measured extreme value corresponds to a maximum or a minimum.

10. The apparatus according to claim 1, wherein the toxin measuring device (13) comprises an optical sensor for measuring an absorption characteristic, in particular the extinction, of the used dialysate.

11. The apparatus according to claim 10, moreover comprising a neuronal network for correcting the conductivity measurement of the first measuring device (8) using a sigmoid activating function, in particular a hyperbolic tangent function, in an intermediate layer.

**Revendications**

1. Dispositif de traitement extracorporel du sang, en particulier pour l'hémodialyse, avec :

- un dialyseur (4), auquel du liquide de dialyse frais peut être amené par le biais d'une conduite d'amenée de liquide de dialyse et duquel du liquide usagé peut être évacué par le biais d'une conduite d'évacuation de liquide de dialyse ;
- une conduite de dérivation qui relie les conduites d'amenée et d'évacuation de liquide de dialyse en contournant le dialyseur (4) et peut être libérée au choix pour un court-circuitage du dialyseur (4) ;
- un premier appareil de mesure (8) pour la mesure d'une grandeur corrélée avec la concentration en sodium dans le plasma d'un sang à nettoyer dans le dialyseur (4), appareil qui est monté directement en aval du dialyseur (4) ;
- une unité de proportionnement (6) pour l'ajustement automatique de la composition du liquide de dialyse en fonction de la grandeur mesurée de telle manière que la concentration en sodium dans le plasma du sang présente au moins à la fin du traitement du sang la même valeur qu'au début, et
- un dispositif de commande (11) pour l'émission d'ordres et la détection au moins des valeurs de mesure ou états de l'appareil de mesure (8), de l'unité de proportionnement (6), et des premières à troisièmes valves (7, 9, 10),
- un appareil de mesure de toxine pour la mesure d'une charge en toxine du liquide de dialyse usagé émis par le dialyseur (4), dans lequel la charge en toxine mesurée peut être utilisé pour la correction d'une mesure de conductivité du premier appareil de mesure (8), **dans lequel**
- les sections de conduite se trouvant entre la conduite de dérivation et le dialyseur (4) des conduites d'amenée et d'évacuation de liquide de dialyse sont maintenues sans pompe et le dispositif de commande (11) est conçu afin qu'au moins au début du traitement extracorporel du sang, du liquide de dialyse reste à l'arrêt dans le dialyseur (4) au moyen d'un mode de fonctionnement de dérivation intermédiaire de sorte qu'à la fin du mode de fonctionnement de dérivation, le dispositif de mesure (8) soit configuré afin de déterminer la grandeur corrélée.

2. Dispositif selon la revendication 1, dans lequel l'unité de proportionnement (6) est conçue de façon à ajuster la composition du liquide de dialyse en fonction de la grandeur mesurée de sorte que la concentration en sodium dans le plasma du sang pendant le traitement du sang soit abaissée, augmentée ou maintenue sensiblement constante.

3. Dispositif selon la revendication 1 ou 2, dans lequel le premier appareil de mesure (8) comprend un appareil de mesure de conductivité, un appareil de mesure spécifique à une substance et/ou un appareil de mesure optique.

4. Dispositif selon la revendication 3, dans lequel le premier appareil de mesure (8) comprend une cellule de conductivité compensant la température.

5. Dispositif selon l'une quelconque des revendications précédentes, dans lequel le dispositif est configuré afin de mettre en place un mode de dérivation, pendant lequel le liquide de dialyse est conduit le long du dialyseur (4) de sorte qu'un volume restant accepte au moins partiellement du côté du liquide de dialyse usagé la concentration des substances dissoutes côté sang, et d'amener au premier appareil de mesure (8) le volume restant du côté du liquide de dialyse usagé pour la mesure de la grandeur corrélée avec la concentration en sodium dans le plasma du sang.

6. Dispositif selon la revendication 5, dans lequel le mode de dérivation est mis en place dans les 20 premières minutes, de préférence dans les 15 premières minutes, encore plus préférentiellement dans les 10 premières minutes, du traitement du sang.

7. Dispositif selon l'une quelconque des revendications précédentes, dans lequel l'unité de proportionnement (6) ajuste la composition du liquide de dialyse de sorte que du sodium soit prélevé du sang dans la mesure où la concentration en sodium dans le plasma vers la fin du traitement du sang corresponde à la concentration en sodium initiale dans le plasma.

8. Dispositif selon la revendication 5 ou 6, dans lequel le premier appareil de mesure (8) est configuré afin de mesurer la conductivité du volume restant sur le côté du liquide de dialyse usagé pour des durées plus longues et plus courtes du mode de dérivation, et dans lequel le dispositif est configuré afin de déterminer un facteur par comparaison des résultats de mesure obtenus, au moyen duquel la conductivité du plasma ou de l'eau du plasma est calculée à l'entrée du dialyseur (4) sur la base de conductivités mesurées du liquide de dialyse usagé avant et après le mode de fonctionnement de dérivation.

9. Dispositif selon l'une quelconque des revendications précédentes, comprenant en outre un second appareil de mesure (12) pour la réalisation d'une seconde mesure de la grandeur corrélée avec la concentration en sodium dans le plasma du sang dans une conduite d'amenée du liquide de dialyse, dans lequel la valeur de mesure du second appareil de mesure est comparée avec la valeur de mesure du premier appareil de mesure (8) afin de constater si une valeur extrême mesurée correspond à un maximum ou un minimum.

10. Dispositif selon la revendication 1, dans lequel l'appareil de mesure de toxine (13) comprend un capteur optique pour la mesure d'une propriété d'absorption, en particulier l'extinction, du liquide de dialyse usagé.

11. Dispositif selon la revendication 10, comprenant en outre un réseau neuronal (21) pour la correction de la mesure de conductivité du premier appareil de mesure (8) en utilisant une fonction d'activation de sigmoïde, en particulier une fonction tangentielle hyperbolique, dans une couche intermédiaire.

**Fig. 1**

**Fig. 2**

**Fig. 3A**

**Fig. 3B**

EP 3 431 118 B1

**Fig. 4**

**Fig. 5**

**Fig. 6**

**Fig. 7**

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017080970 A1
- DE 19734992 C1
- EP 0407737 A1

**In der Beschreibung aufgeführte Nicht-Patentliteratur**

- **PAULA, F. M. ; PEIXOTO, A. J. ; PINTO, I. V ; DORIGO, D. ; PATRICIO, P. J. M ; SANTOS, S. F. F.** Clinical consequences of an Individualized dialysate sodium Prescription in hemodialysis patients. *Kidney Internationol,* 2004, vol. 66, 1232-1238
- **BASILE, C. ; LOMONTE. C.** It is Time to Individualize the Dialysate Sodium Prescription. *Seminars in Dialysis,* 2016, vol. 29, 24-27
- **MENDOZA, J. M ; SUN, S. ; CHERTOW, G. M ; MORAN, J ; DOSS, S. ; SCHILLER, B.** Dialysate Sodium and Sodium gradient in maintenance hemodialysis: a neglected sodium restriction approach?. *Nephrol Dial Transplant.,* 2011, vol. 26, 1281-1287